# EUROPEAN PATENT APPLICATION

(11) **EP 4 070 825 A1**
(43) Date of publication of application: **12.10.2022**
(21) Application number: 20904609.3
(22) Date of filing: 25.11.2020
(51) Int. Cl.: A61L 31/10, A61L 31/16

(54) **DRUG-LOADED IMPLANTABLE MEDICAL INSTRUMENT AND MANUFACTURING METHOD THEREFOR**

(30) Priority: 27.12.2019 CN 201911380555
(71) Applicant: Shanghai MicroPort Medical (Group) Co., Ltd., Shanghai 201203 (CN)
(72) Inventor: LI, Meng, Shanghai 201203 (CN); LI, Junfei, Shanghai 201203 (CN); WANG, Yufu, Shanghai 201203 (CN)
(74) Representative: Arnold & Siedsma
(86) International application number: PCT/CN2020/131333
(87) International publication number: WO 2021/129279

(57) **Abstract**

Provided are a drug-loaded implantable medical instrument and a manufacturing method therefor. The drug-loaded implantable medical instrument (10) comprises an instrument body (100), a microporous membrane (200) fixed on the instrument body (100), and a nanocrystal medicament (300) loaded on the microporous membrane (200).

## Description

### CROSS REFERENCE TO RELATED APPLICATION

The present application claims priority to Chinese Patent Application No. 2019113805552, entitled "DRUG-LOADED IMPLANTABLE MEDICAL DEVICE AND MANUFACTURING METHOD THEREOF", filed on December 27, 2019, the content of which is incorporated herein by reference in its entirety.

### TECHNICAL FIELD

The present application relates to the field of medical devices, and more particularly, to a drug-loaded implantable medical device and a method for preparing the drug-loaded implantable medical device.

### BACKGROUND

Currently, with the development of social economy, the national life style has changed deeply. In particular, with the acceleration of the aging of the population and the urbanization process, the epidemic trend of risk factors for cardiovascular disease in China is obvious, resulting in a continuous increase in the number of patients with cardiovascular disease, resulting in a continuous increase in the number of patients with cardiovascular disease. Therefore, the prevention and treatment of cardiovascular diseases has increasingly become the focus of attention of doctors around the world.

Since the 1970s, the treatment of various cardiovascular diseases by interventional medical devices has become increasingly common. It has experienced three milestones of rapid development: simple balloon dilatation (PTCA), bare metal stent (BMS), and drug eluting stent (DES). In particular, drug-eluting stents have achieved great success in the treatment of vascular stenosis, showing the potential of DES in the treatment of stenosis. However, the drug-eluting stents still have the following disadvantages: (1) a restenosis rate of about 5% still occurs, which is more and more unnegligible as the number of percutaneous coronary intervention (PCI) surgeries continues to increase; (2) the polymer coating matrix of the drug-eluting stents may induce inflammatory responses and delay wound healing, and the coating drugs may inhibit a proliferation of smooth muscle cells and also inhibit a regeneration of endothelial cells, resulting in a delay in an endothelialization process of vascular after the stent is implanted; and (3) the drug-eluting stents are difficult to applied to in-stent restenosis, small vessel disease and bifurcation disease; moreover, due to the need to take double antibody for a long time, the application to patients who are prone to bleeding is limited. In this case, a drug coated balloon (DCB) is developed to provide a new option for the treatment of the above situation, and to provide a new hope for the long-term prognosis of the interventional treatment of coronary heart disease. A surface of the drug coated balloon is uniformly coated with anti-hyperplasia drugs, and then, the drug coated balloon is delivered to a lesion site and releases drugs within a short expansion time (in a range from 30s to 60s) to inhibit the hyperplasia of vascular smooth muscle cells. Due to the advantages of drug coated balloon, such as no implantation during intervention, no risk of thrombosis, and rapid treatment effect, the drug coated balloon has attracted more and more attention.

The anti-hyperplasia drugs on the surface of the drug coated balloon are mainly in amorphous and crystalline states. It has been found that when the drugs are present in the amorphous state in a drug coating of drug balloon, the drug coating is relatively uniform, the particles formed in the drug release process are relatively small, the risk of embolization is relatively small, and the safety is relatively high. However, the drags with the amorphous state have a poor retention effect in tissues, generally within a week, concentrations of the drugs in the tissues has dropped below a therapeutic concentration, so that it is difficult to effectively inhibit the hyperplasia of vascular smooth muscle cells. On the other hand, although the drugs with the crystalline state have an excellent slow-release effect and a long retention time in tissues, an uniformity of the surface of the drug coating also becomes very poor, it is easier to form particles with a larger size, and thus it is easy to cause terminal embolization. Moreover, the drug with the crystalline state and a large size may cause a high local concentration of the drug, resulting in a high risk of toxicity and side effects.

### SUMMARY

According to various embodiments of the present application, a drug-loaded implantable medical device and a method for preparing the drug-loaded implantable medical device are provided.

A drug-loaded implantable medical device includes a device body, a microporous membrane fixed on the device body, and a nanocrystalline drug loaded on a surface of the microporous membrane.

In an embodiment, the surface of the microporous membrane and the nanocrystalline drug are charged, and a charge on the surface of the microporous membrane is of the same type as a charge on the nanocrystalline drug.

In an embodiment, the microporous membrane is formed from at least one of nylon, polyvinylidene fluoride, mixed cellulose, polytetrafluoroethylene, polypropylene, polyethersulfone, or glass fibers.

In an embodiment, the microporous membrane has a porosity in a range from 40% to 90%.

In an embodiment, the microporous membrane has a pore size in a range from 0.02µm to 0.8µm.

In an embodiment, the microporous membrane has a thickness in a range from 1µm to 200µm.

In an embodiment, the drug-loaded implantable medical device further includes a stabilizer adsorbed on a surface of the nanocrystalline drug, and the mass of the stabilizer is in a range from 0.2% to 20% of a total mass of the nanocrystalline drug.

In an embodiment, the stabilizer is selected from one or more of poloxamer, polyvinylpyrrolidone (PVP), tween, hydroxypropyl methyl cellulose (HPMC), dextran, sodium dodecyl sulfate (SDS), sodium carboxymethylcellulose and polyvinyl alcohol (PVA).

In an embodiment, the nanocrystalline drug is an anti-hyperplasia drug.

In an embodiment, the nanocrystalline drug has a particle size in a range from 20nm to 300nm.

In an embodiment, the nanocrystalline drug has a spherical, rod-like, worm-like or disk-like morphology.

In an embodiment, in the nanocrystalline drug, the mass percentage of crystalline drugs is in a range from 70% to 100%.

In an embodiment, the device body is a balloon.

A method for preparing a drug-loaded implantable medical device includes:
providing a microporous membrane;
loading a nanocrystalline drug on the microporous membrane; and
fixing the microporous membrane loaded with the nanocrystalline drug to the device body.

In an embodiment, the nanocrystalline drug is loaded on the microporous membrane by a mechanical filtration.

In an embodiment, the microporous membrane loaded with the nanocrystalline drug is fixed to the device body by a laser welding.

In an embodiment, the loading the nanocrystalline drug on the microporous membrane includes:
dissolving a drug in a first solvent to obtain a drug solution;
suspending a stabilizer in a second solvent to obtain a stabilizer suspension;
adding the drug solution to the stabilizer suspension under stirring to obtain a mixed solution;
sonicating, and then dialyzing, concentrating the mixed solution to obtain a nanocrystalline drug suspension; and
loading the nanocrystalline drug in the nanocrystalline drug suspension on the microporous membrane, and then drying.

One of the first solvent and the second solvent is an organic solvent that is miscible with water, and the other is water.

The above drug-loaded implantable medical device innovatively uses a manner in which the microporous membrane is loaded with the nanocrystalline drug, so that the nanocrystalline drug is firmly loaded on the microporous membrane, and is not easy to fall off during delivery. When the target lesion is reached, the nanocrystalline drug is redissolved and dispersed due to an expansion effect of the device itself and a dissolution effect of the blood, thereby improving an utilization rate of drugs. In addition, compared with conventional nano-drugs with core-shell structure, the microporous membrane is loaded with the nanocrystalline drug, which may greatly increases the drug loading amount, and avoid the use of a large number of excipients, carriers, and the like, and thus improve the safety; which is also beneficial to reduce the size of the nanocrystalline drug and avoids the risk of embolism and the toxic side effects; and which may further effectively increase the content of the crystalline drug in the nanocrystalline drug, thereby enhancing the slow-release effect and prolonging the retention time in tissues.

### BRIEF DESCRIPTION OF THE DRAWINGS

To illustrate the solutions of the embodiments of the present application or of the prior art more clearly, the accompany drawings for describing the embodiments or the prior art are introduced briefly in the following. Apparently, the accompanying drawings in the following description are only some embodiments of the present application, and persons of ordinary skill in the art can derive accompany drawings of the other embodiments from these accompanying drawings without any creative efforts.
FIG. 1 is a schematic view of a drug-loaded implantable medical device according to an embodiment.
FIG. 2 illustrates a morphology of a microporous membrane made of nylon, magnified 10,000 times under electron microscope.
FIG. 3 illustrates a morphology of a microporous membrane made of polytetrafluoroethylene (PTFE), magnified 5,000 times under electron microscope.
FIG. 4 illustrates a morphology of a microporous membrane made of polyvinylidene fluoride (PVDF), magnified 10,000 times under electron microscope.
FIG. 5 is a flowchart illustrating a method for preparing the drug-loaded implantable medical device according to an embodiment.
FIG. 6 is a graph illustrating a size distribution of a nanocrystalline drug prepared in Example 1.
FIG. 7 is an XRD pattern of the nanocrystalline drug prepared in Example 1.

### DETAILED DESCRIPTION OF THE EMBODIMENTS

In order to facilitate the understanding of the present application, the present application will be described more fully hereinafter, and preferred embodiments of the present application are given below. However, the present application may be embodied in many different forms and is not limited to the embodiments described herein. Rather, these embodiments are provided so that the understanding of the content of the present application will be more thorough.

All technical and scientific terms used herein have the same meaning as commonly understood by one of ordinary skill in the art to which the present application applies, unless otherwise defined. The terms used in the specification of present application herein are for the purpose of describing specific embodiments only and are not intended to limit the present application. The term "and/or" used herein includes any and all combinations of one or more of the associated listed items.

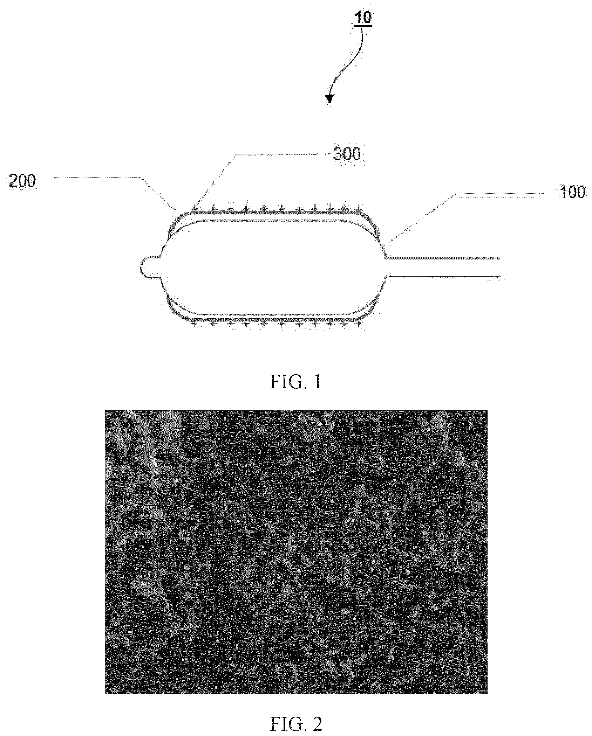

As shown in FIG. 1, according to an embodiment, a drug-loaded implantable medical device 10 is provided, which includes a device body 100, a microporous membrane 200 fixed on the device body 100, and a nanocrystalline drug 300 loaded on the microporous membrane 200.

It could be appreciated that the above drug-loaded implantable medical device 10 may be used in vivo or in vitro, for short-term use or for long-term permanent implantation. Further, the above medical device may be an device that provides medical treatment and/or diagnosis for cardiac rhythm disorders, heart failure, valvular diseases, vascular diseases, diabetes mellitus, neurological diseases and disorders, plastic surgery, neurosurgery, oncology, ophthalmology, and ENT surgery. The medical devices involved in the present application includes, but are not limited to the following devices, such as, stents, stent-grafts, anastomotic connectors, synthetic patches, leads, electrodes, needles, wires, catheters, sensors, surgical devices, angioplasty balls, wound drainage tubes, shunts, tubes, infusion sleeves, urethral catheters, pellets, implants, blood oxygenation generators, pumps, vascular grafts, vascular access port, heart valves, annuloplasty rings, sutures, surgical clips, surgical staples, pacemakers, implantable defibrillators, neurostimulators, plastic surgical devices, cerebrospinal fluid shunts, implantable medication pumps, vertebral cages, artificial discs, replacement devices for the nucleus pulposus, ear tubes, intraocular lenses and any tubes used in interventional operations. The stents include, but are not limited to, coronary vascular stents, peripheral vascular stents, intracranial vascular stents, urethral stents, and esophageal stents. In the present embodiment, the above drug-loaded implantable medical device is a drug-coated balloon, i.e., a device body is a balloon.

In an embodiment, the microporous membrane 200 refers to a membrane with a network structure containing micropores, and a pore size of the micropores may be adjusted according to a particle size of the nanocrystalline drug. Therefore, the size of the nanocrystalline drug loaded on the microporous membrane may be adjusted by selecting a microporous membrane with a suitable pore size.

In an embodiment, the nanocrystalline drug 300 has a particle size greater than or equal to the pore size of the microporous membrane 200, so that it is easier to load the nanocrystalline drug 300 on the microporous membrane 200 by a mechanical filtration. In an embodiment, the pore size of the microporous membrane 200 is in a range from 0.02µm to 0.8µm. Further, the pore size of the microporous membrane 200 may be in a range from 0.1µm to 0.5µm. Further, the pore size of the microporous membrane 200 may be in a range from 0.1µm to 0.3µm. In an embodiment, the microporous membrane 200 has a porosity in a range from 40% to 90%. The porosity refers to the percentage of a pore volume in a material to a total volume of the material in a natural state. In an embodiment, the microporous membrane 200 has a thickness in a range from 1µm to 200µm. Further, the microporous membrane 200 has a thickness in a range from 1µm to 50µm.

In an embodiment, the microporous membrane 200 may be formed from one or more of following materials: nylon, polyvinylidene fluoride, mixed cellulose, polytetrafluoroethylene, polypropylene, polyethersulfone, or glass fibers. In an embodiment, the microporous membrane 200 is formed of a nylon material (e.g., nylon 66, etc.), which may improve a loading efficiency and facilitate the nanocrystalline drug 300 to be firmly loaded on the microporous membrane 200.

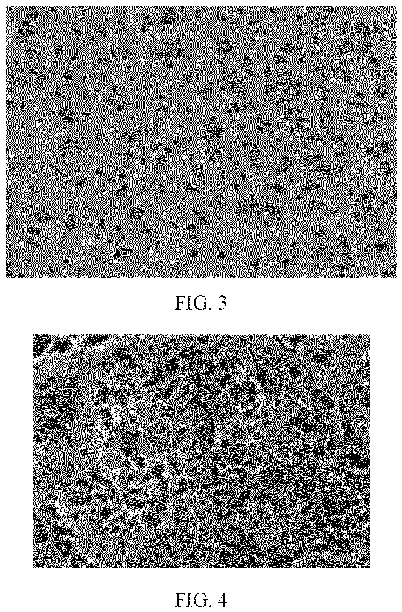

FIG. 2 illustrates a morphology of a microporous membrane 200 made of nylon, magnified 10,000 times under electron microscope. FIG. 3 illustrates a morphology of a microporous membrane 200 made of PTFE, magnified 5,000 times under electron microscope. FIG. 4 illustrates a morphology of a microporous membrane 200 made of PVDF, magnified 10,000 times under electron microscope. As may be seen from FIG. 2 to FIG. 4, the microporous membranes 200 have a relatively high porosity, and the pores are formed uniformly, which are suitable for loading the nanocrystalline drug 300. It will be appreciated that the material and type of the microporous membrane 200 are not limited thereto, and other membrane with higher porosity, such as nylon transfer membranes for transfer and detection of proteins and nucleic acids, or the like, may be used.

The nanocrystalline drug 300 may be loaded on a side of the microporous membrane 200 away from the device body 100. In an embodiment, a surface of the microporous membrane 200 and the nanocrystalline drug 300 are charged, and the charge on the surface of the microporous membrane 200 is of the same type as the charge on the nanocrystalline drug 300. In this way, by utilizing a repulsion effect of the charges, the release of the nanocrystalline drug 300 can be greatly promoted, so that the drug utilization rate is high, and the required drug dose is low.

In an embodiment, the nanocrystalline drug 300 refers to a nano-sized (less than 1000 nm) drug crystal. It will be appreciated that the nanocrystalline drug 300 may include a crystalline drug and an amorphous drug. In an embodiment, in the nanocrystalline drug 300, a mass percentage of the crystalline drug may be in a range from 0% to 100%, and a mass percentage is in a range from 70% to 100%.

In an embodiment, the nanocrystalline drug 300 has a drug loading amount in a range from 1% to 99%, and further, in a range from 50% to 100%.

In an embodiment, the nanocrystalline drug 300 has a particle size in a range from 1nm to 1000nm, and further, in a range from 3nm to 300nm. Further, the nanocrystalline drug 300 has a particle size in a range from 20nm to 300nm, and further, in a range from 50nm to 250nm.

In an embodiment, the nanocrystalline drug 300 may have a spherical, rod-like, worm-like or disk-like morphology.

In an embodiment, the drug-loaded implantable medical device 10 may further include a stabilizer adsorbed on the surface of the nanocrystalline drug 300. In an embodiment, the mass of the stabilizer is in a range from 0.2% to 20% of the total mass of the nanocrystalline drug. A small amount of stabilizer is adsorbed on the surface of the nanocrystalline drug 300, which may increase the stability of the nanocrystalline drug 300 and avoid the phenomenon, such as nanoparticle agglomeration, etc., thereby facilitating the formation of the nanocrystalline drug 300 with a smaller size. In addition, compared with the conventional nano-drugs with core-shell structure, the above nanocrystalline drug 300 may greatly increase the drug loading amount (the maximum drug loading amount may be close to 100%), and it is more convenient to adjust the particle size of the nanocrystalline drug. In an embodiment, the stabilizer is selected from at least one of poloxamer, polyvinylpyrrolidone (PVP), tween, hydroxypropyl methyl cellulose (HPMC), dextran, sodium dodecyl sulfate (SDS), sodium carboxymethylcellulose and polyvinyl alcohol (PVA).

The nanocrystalline drug 300 may be selected according to actual needs. For example, the nanocrystalline drug 300 may be an anti-proliferative, anti-inflammatory, antiphlogistic, anti-hyperplasia, anti-bacterial, anti-tumor, anti-mitotic, cell-suppressing, cytotoxic, anti-osteoporotic, anti-angiogenic, anti-restenosis, microtubule-inhibiting, anti-metastatic or anti-thrombotic drug. The nanocrystalline drug 300 include, but are not limited to, dexamethasone, prednisolone, corticosterone, budesonide, estrogen, sulfasalazine and aminosalicylic acid, acemetacin, aescinate, aminopterin, antimycoin, arsenic trioxide, aristolochic acid, aspirin, berberine, bilobol, rapamycin and derivatives thereof (including zotarolimus, everolimus, biomos, 7-O-desmethyl rapamycin, temsirolimus, ridaforolimus, etc.), endothelial statin, angiostatin, angipoietin, monoclonal antibodies capable of blocking the proliferation of smooth muscle cells, levofloxacin, paclitaxel, docetaxel, hydroxycamptothecine, vinblastine, vincristine, doxorubicin, 5-fluorouracil, cisplatin, thymidine kinase inhibitor antibiotics (especially actinomycin-D), hormones, antibody cancer drugs, bisphosphonates, selective estrogen receptor modulators, strontium ranelate, cyclosporine A, cyclosporine C, and brefeldin A.

In an embodiment, the nanocrystalline drug 300 is an anti-hyperplasia drug. Further, the nanocrystalline drug 300 may be paclitaxel, a paclitaxel derivative, rapamycin, or a rapamycin derivative. In an embodiment, the rapamycin derivative may be everolimus or zotarolimus.

In the above drug-loaded implantable medical device 10, innovatively, the microporous membrane 200 is used to be loaded with the nanocrystalline drug 300, so that the nanocrystalline drug 300 is firmly loaded on the microporous membrane 200, which ensures that the nanocrystalline drug 300 is not easy to fall off from the microporous membrane 200 during delivery. When the nanocrystalline drug 300 reaches the target lesion, the nanocrystalline drug 300 is redissolved and dispersed due to an expansion effect of the drug-loaded implantable medical device 10 itself and a dissolution effect of the blood, thereby improving an utilization rate of the drug. In addition, compared with the conventional nano-drugs with core-shell structure, since the microporous membrane 200 is loaded with the nanocrystalline drug 300, the drug loading amount is greatly increased, and the use of a large number of adjuvant carriers and the like is avoided, and thus the safety is improved. Further, it may be beneficial to reduce the size of the nanocrystalline drug 300 to avoid the risk of embolization and toxic side effects. Further, the content of the crystalline drug in the nanocrystalline drug 300 may be effectively increased, thereby improving the slow-release effect and prolonging the retention time in tissues.

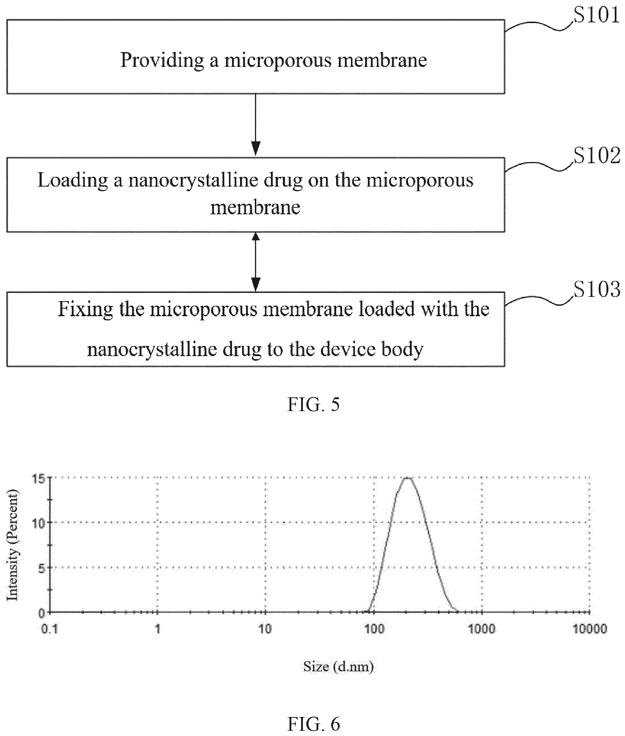

Referring to FIG. 5, a method for preparing the drug-loaded implantable medical device according to an embodiment includes the following steps.

In step S101, a microporous membrane is provided.

In step S101, the selection of the microporous membrane is as described above, which is not repeated here.

In step 102, a nanocrystalline drug is loaded on the microporous membrane.

In an embodiment, the nanocrystalline drug is loaded on the microporous membrane by a mechanical filtration. The mechanical filtration is simple and fast to operate, which avoids the expensive and time-consuming ultrasonic spraying procedures commonly used in the industry, thereby simplifying the large-scale preparation and having great industrialization prospects. The nanocrystalline drug may be tightly attached to the microporous membrane by the mechanical filtration, so that the nanocrystalline drug is firmly attached to the microporous membrane and is not easy to fall off from the microporous membrane during delivery.

In an embodiment, step 102 includes the following steps. In step (a), the drug is dissolved in a first solvent to obtain a drug solution, and a stabilizer is suspended in a second solvent to obtain a stabilizer suspension. The first solvent and the second solvent may be selected depending on the kinds of drug and stabilizer. In an embodiment, the first solvent is an organic solvent that is miscible with water, and the second solvent is water. Of course, vice versa. Further, the concentration of the drug solution is in a range from 20mg/mL to 60mg/mL; and the concentration of stabilizer in the stabilizer suspension is in a range from 0.05% to 0.3%. In step (b), the drug solution is added to the stabilizer suspension under stirring to obtain a mixed solution. The drug solution is slowly added to the stabilizer suspension, and the drug is gradually precipitated out according to a principle of reverse solvent. Moreover, the stabilizer in the suspension may cause that when the drug is precipitated out, a small amount of the stabilizer is adsorbed on the surface of the drug, so that the agglomeration of the nanoparticles may be effectively avoided, which is conducive to form precipitated particles with small particle size and the crystalline drug.

In addition, the method in step (b) is used to form a particle morphology in which pure nano-drug particles adsorb a small amount of the stabilizer, which not only has high stability but also has a higher drug loading amount than that of the conventional nano-drugs with core-shell structure, and may further avoid the addition of a large amount of carriers, excipients, and the like, and reduce the toxic and side effects.

In step (c), the mixed solution is sonicated, and then dialyzed and concentrated to obtain a nanocrystalline drug suspension.

Sonicating the mixed solution may facilitate the formation of the nanocrystalline drug with small-particles and the transformation from the amorphous drug to the crystalline drug, increase the proportion of crystalline drugs in the nanocrystalline drug, and thus improve the slow-release effect of drug-loaded implantable medical device.

In step (c), probe-type ultrasound (also called ultrasonic cell pulverizer) or water-bath ultrasound (also called ultrasonic cleaner) may be used to perform the sonicating. A sonicating time may be adjusted as desired. In an embodiment, the sonicating time is in a range from 15min to 30min. After performing the sonicating, the mixed solution may be placed into a dialysis bag for dialysis, and water is changed every once in a while to prepare and obtain the nanocrystalline drug suspension. Finally, the prepared nanocrystalline drug suspension is concentrated for subsequent use.

In step (d), the nanocrystalline drug in the nanocrystalline drug suspension is loaded on the microporous membrane, and then drying is performed.

In an embodiment, one of surfaces of the microporous membrane is loaded with the nanocrystalline drug, and the other surface of the microporous membrane that is not loaded with the nanocrystalline drug is attached to the device body to facilitate the fixation of the microporous membrane. Further, in step (d), the microporous membrane may be first fixed to a filter mold, and then an appropriate amount of the above nanocrystalline drug suspension may be extracted by a syringe or the like, and injected onto the microporous membrane, so that the nanocrystalline drug is loaded on the microporous membrane, and then drying is performed.

In step S103, the microporous membrane loaded with the nanocrystalline drug is fixed to the device body.

In step S103, the microporous membrane loaded with the nanocrystalline drug may be fixed to the device body by laser welding, so as to improve the attaching strength of the microporous membrane and the device body, and to prevent the microporous membrane from falling off. The method is relatively simple and suitable for mass production.

The above method for preparing the drug-loaded implantable medical device has the following advantages.

(1) Innovatively, nanotechnology is combined with crystallization technology to obtain the nanocrystalline drug with smaller size, reducing embolization and toxic side effects. Further, the above nanocrystalline drug is a pure drug crystal, only a small amount of stabilizer is adsorbed on the surface, and the drug loading amount may be close to 100% maximally. Unlike the conventional nano-drugs with core-shell structure in which the drug is coated on the carrier, the above nanocrystalline drugs may greatly increase the content of the crystalline drug, thereby obtaining an excellent slow-release effect and a long retention time in tissues. Moreover, the nano-sized nanocrystalline drug may avoid the risk of embolization and the toxic side effects, which is safe and effective.

(2) Innovatively, the microporous membrane is used, and the nanocrystalline drug is loaded on the surface of the microporous membrane firmly and stably, so that the nanocrystalline drug is not easy to fall off during delivery. When the target lesion is reached, the nanocrystalline drug is redissolved and dispersed due to an effect of the device itself and a dissolution effect of the blood. In addition, the microporous membrane and the nanocrystalline drug may be charged with the same charge, so that on the basis of the repulsion between the charges of the microporous membrane and the charges of the nanocrystalline drug, the release of the nanocrystalline drug may be further improved, and the utilization rate of the drug may be further improved, and the required drug dose is reduced.

(3) The adjustability is high, for example, the concentration of the nanocrystalline drug suspension and the pore size of the microporous membrane may be adjusted as needed, so as to adjust the target drug loading amount; the appropriate microporous membrane may also be selected according to the needs, so as to adjust the particle size of the nanocrystalline drug; and the microporous membrane and nanocrystalline drug with suitable charges may be selected according to the needs, so as to utilize the effect of charges to control the release rate of the nanocrystalline drug particles.

The present application will be described hereinafter in combination with the specific embodiments.

### Example 1

Poloxamer 188 was dissolved thoroughly in pure water to obtain an aqueous solution of poloxamer at a concentration of 0.15% (w/v). Rapamycin was dissolved in acetone to obtain a acetone solution of rapamycin at a concentration of 40mg/mL. The above acetone solution of rapamycin was slowly added to the above aqueous solution of poloxamer under stirring to obtain a mixed solution. Subsequently, the mixed solution was transferred to an ultrasonic cell pulverizer and was sonicated for 20 minutes, after that, the mixed solution was placed into a dialysis bag for dialysis for 12h, and the water was changed every 2h to prepare a nanocrystalline drug suspension. Subsequently, the prepared nanocrystalline drug suspension was concentrated for subsequent use. The size and surface charge of the nanocrystalline drug were characterized by Malvern ZS90 tests. The drug loading amount of the nanocrystalline drug was calculated by high performance liquid chromatography (HPLC). The crystalline form of the nanocrystalline drug was detected by X-ray powder diffraction (XRPD).

A piece of nylon microporous membrane having a negative surface charge and a pore size of 0.22µm was sandwiched on a filter mold. The above nanocrystalline drug suspension with the appropriate concentration was extracted by a syringe, was loaded on the nylon microporous membrane, and was vacuum-dried overnight. Subsequently, the nylon microporous membrane loaded with the nanocrystalline drug was tightly welded to the surface of a conventional balloon by laser welding, and the sterilization was performed with ethylene oxide, thereby obtaining the drug-loaded implantable medical device (i.e., the drug coated balloon).

### Example 2

Polyvinylpyrrolidone K30 was dissolved thoroughly in pure water to obtain an aqueous solution of polyvinylpyrrolidone at a concentration of 0.15% (w/v). Rapamycin was dissolved in acetone to obtain a acetone solution of rapamycin at a concentration of 40mg/mL. The above acetone solution of rapamycin was slowly added to the above aqueous solution of polyvinylpyrrolidone under stirring to obtain a mixed solution. Subsequently, the mixed solution was transferred to an ultrasonic cell pulverizer and was sonicated for 20 minutes, after that, the mixed solution was placed into a dialysis bag for dialysis for 12h, and the water was changed every 2h to prepare a nanocrystalline drug suspension. Subsequently, the prepared nanocrystalline drug suspension was concentrated for subsequent use. The size and surface charge of the nanocrystalline drug were characterized by Malvern ZS90 tests. The drug loading amount of the nanocrystalline drug was calculated by high performance liquid chromatography (HPLC). The crystalline form of the nanocrystalline drug was detected by X-ray powder diffraction (XRPD).

A piece of nylon microporous membrane having a negative surface charge and a pore size of 0.22µm was sandwiched on a filter mold. The above nanocrystalline drug suspension with the appropriate concentration was extracted by a syringe, was loaded on the nylon microporous membrane, and was vacuum-dried overnight. Subsequently, the nylon microporous membrane loaded with the nanocrystalline drug was tightly welded to the surface of a conventional balloon by laser welding, and the sterilization was performed with ethylene oxide, thereby obtaining the drug-loaded implantable medical device (i.e., the drug coated balloon).

### Example 3

Tween 80 was dissolved thoroughly in pure water to obtain an aqueous solution of tween at a concentration of 0.15% (w/v). Rapamycin was dissolved in acetone to obtain a acetone solution of rapamycin at a concentration of 40mg/mL. The above acetone solution of rapamycin was slowly added to the above aqueous solution of tween under stirring to obtain a mixed solution. Subsequently, the mixed solution was transferred to an ultrasonic cell pulverizer and was sonicated for 20 minutes, after that, the mixed solution was placed into a dialysis bag for dialysis for 12h, and the water was changed every 2h to prepare a nanocrystalline drug suspension. Subsequently, the prepared nanocrystalline drug suspension was concentrated for subsequent use. The size and surface charge of the nanocrystalline drug were characterized by Malvern ZS90 tests. The drug loading amount of the nanocrystalline drug was calculated by high performance liquid chromatography (HPLC). The crystalline form of the nanocrystalline drug was detected by X-ray powder diffraction (XRPD).

A piece of nylon microporous membrane having a negative surface charge and a pore size of 0.22µm was sandwiched on a filter mold. The above nanocrystalline drug suspension with the appropriate concentration was extracted by a syringe, was loaded on the nylon microporous membrane, and was vacuum-dried overnight. Subsequently, the nylon microporous membrane loaded with the nanocrystalline drug was tightly welded to the surface of a conventional balloon by laser welding, and the sterilization was performed with ethylene oxide, thereby obtaining the drug-loaded implantable medical device (i.e., the drug coated balloon).

### Example 4

HPMC E5 was dissolved thoroughly in pure water to obtain an aqueous solution of HPMC E5 at a concentration of 0.15% (w/v). Rapamycin was dissolved in acetone to obtain a acetone solution of rapamycin at a concentration of 40mg/mL. The above acetone solution of rapamycin was slowly added to the above aqueous solution of HPMC E5 under stirring to obtain a mixed solution. Subsequently, the mixed solution was transferred to an ultrasonic cell pulverizer and was sonicated for 20 minutes, after that, the mixed solution was placed into a dialysis bag for dialysis for 12h, and the water was changed every 2h to prepare a nanocrystalline drug suspension. Subsequently, the prepared nanocrystalline drug suspension was concentrated for subsequent use. The size and surface charge of the nanocrystalline drug were characterized by Malvern ZS90 tests. The drug loading amount of the nanocrystalline drug was calculated by high performance liquid chromatography (HPLC). The crystalline form of the nanocrystalline drug was detected by X-ray powder diffraction (XRPD).

A piece of nylon microporous membrane having a negative surface charge and a pore size of 0.22µm was sandwiched on a filter mold. The above nanocrystalline drug suspension with the appropriate concentration was extracted by a syringe, was loaded on the nylon microporous membrane, and was vacuum-dried overnight. Subsequently, the nylon microporous membrane loaded with the nanocrystalline drug was tightly welded to the surface of a conventional balloon by laser welding, and the sterilization was performed with ethylene oxide, thereby obtaining the drug-loaded implantable medical device (i.e., the drug coated balloon).

### Example 5

Poloxamer 188 was dissolved thoroughly in pure water to obtain an aqueous solution of poloxamer at a concentration of 0.15% (w/v). Everolimus was dissolved in acetone to obtain an acetone solution of everolimus at a concentration of 50mg/mL. The above acetone solution of everolimus was slowly added to the above aqueous solution of poloxamer under stirring to obtain a mixed solution. Subsequently, the mixed solution was transferred to an ultrasonic cell pulverizer and was sonicated for 20 minutes, after that, the mixed solution was placed into a dialysis bag for dialysis for 12h, and the water was changed every 2h to prepare a nanocrystalline drug suspension. Subsequently, the prepared nanocrystalline drug suspension was concentrated for subsequent use. The size and surface charge of the nanocrystalline drug were characterized by Malvern ZS90 tests. The drug loading amount of the nanocrystalline drug was calculated by high performance liquid chromatography (HPLC). The crystalline form of the nanocrystalline drug was detected by X-ray powder diffraction (XRPD).

A piece of nylon microporous membrane having a negative surface charge and a pore size of 0.22µm was sandwiched on a filter mold. The above nanocrystalline drug suspension with the appropriate concentration was extracted by a syringe, was loaded on the nylon microporous membrane, and was vacuum-dried overnight. Subsequently, the nylon microporous membrane loaded with the nanocrystalline drug was tightly welded to the surface of a conventional balloon by laser welding, and the sterilization was performed with ethylene oxide, thereby obtaining the drug-loaded implantable medical device (i.e., the drug coated balloon).

### Example 6

Hydroxypropyltrimethyl ammonium chloride chitosan was thoroughly dissolved in pure water to obtain an aqueous solution of hydroxypropyltrimethyl ammonium chloride chitosan at a concentration of 0.15% (w/v). Rapamycin was dissolved in acetone to obtain a acetone solution of rapamycin at a concentration of 40mg/mL. The above acetone solution of rapamycin was slowly added to the above aqueous solution of hydroxypropyltrimethyl ammonium chloride chitosan under stirring to obtain a mixed solution. Subsequently, the mixed solution was sonicated with water-bath ultrasound for 20 minutes, after that, the mixed solution was placed into a dialysis bag for dialysis for 12h, and the water was changed every 2h to prepare a nanocrystalline drug suspension. Subsequently, the prepared nanocrystalline drug suspension was concentrated for subsequent use. The size and surface charge of the nanocrystalline drug were characterized by Malvern ZS90 tests. The drug loading amount of the nanocrystalline drug was calculated by high performance liquid chromatography (HPLC). The crystalline form of the nanocrystalline drug was detected by X-ray powder diffraction (XRPD).

A piece of nylon microporous membrane having a positive surface charge and a pore size of 0.22µm was sandwiched on a filter mold. The above nanocrystalline drug suspension with the appropriate concentration was extracted by a syringe, was loaded on the nylon microporous membrane, and was vacuum-dried overnight. Subsequently, the nylon microporous membrane loaded with the nanocrystalline drug was tightly welded to the surface of a conventional balloon by laser welding, and the sterilization was performed with ethylene oxide, thereby obtaining the drug-loaded implantable medical device (i.e., the drug coated balloon).

### Example 7

A piece of uncharged (neutral) nylon microporous membrane having a pore size of 0.22µm was sandwiched on a filter mold. The nanocrystalline drug suspension with the appropriate concentration prepared in the above Example 1 was extracted by a syringe, was loaded on the nylon microporous membrane, and was vacuum-dried overnight. Subsequently, the nylon microporous membrane loaded with the nanocrystalline drug was tightly welded to the surface of a conventional balloon by laser welding, and the sterilization was performed with ethylene oxide, thereby obtaining a drug-loaded implantable medical device (i.e., the drug coated balloon).

### Example 8

Poloxamer 407 was dissolved thoroughly in pure water to obtain an aqueous solution of poloxamer at a concentration of 0.15% (w/v). Rapamycin was dissolved in acetone to obtain a acetone solution of rapamycin at a concentration of 40mg/mL. The above acetone solution of rapamycin was slowly added to the above aqueous solution of poloxamer under stirring to obtain a mixed solution. Subsequently, the mixed solution was transferred to an ultrasonic cell pulverizer and was sonicated for 20 minutes, after that, the mixed solution was placed into a dialysis bag for dialysis for 12h, and the water was changed every 2h to prepare a nanocrystalline drug suspension. Subsequently, the prepared nanocrystalline drug suspension was concentrated for subsequent use. The size and surface charge of the nanocrystalline drug were characterized by Malvern ZS90 tests. The drug loading amount of the nanocrystalline drug was calculated by high performance liquid chromatography (HPLC). The crystalline form of the nanocrystalline drug was detected by X-ray powder diffraction (XRPD).

A piece of nylon microporous membrane having a negative surface charge and a pore size of 0.22µm was sandwiched on a filter mold. The above nanocrystalline drug suspension with the appropriate concentration was extracted by a syringe, was loaded on the nylon microporous membrane, and was vacuum-dried overnight. Subsequently, the nylon microporous membrane loaded with the nanocrystalline drug was sutured on the surface of a cobalt-chromium alloy stent by suturing, and the sterilization was performed with ethylene oxide, thereby obtaining a drug-loaded implantable medical device (i.e., a drug-containing covered stent).

### Comparative Example 1

The nanocrystalline drug suspension prepared in Example 1 was sprayed onto the surface of a conventional balloon (without welding nylon microporous membrane) by ultrasonic spraying, dried overnight, and sterilized with ethylene oxide, so as to obtain a drug coated balloon.

### Performance Characterization

The prepared nanocrystalline drug suspensions loaded on the microporous membranes of Examples 1 to 8 and Comparative Example 1 were characterized, and the test results were shown in Table 1.

### Test method:

Particle size: Photon correlation spectroscopy, with a device of Malvern Zetasizer Nano ZS90; Polydispersity index: Photon correlation spectroscopy, with a device of Malvern Zetasizer Nano ZS90; Surface charge: Photon correlation spectroscopy, with a device of Malvern Zetasizer Nano ZS90; and

Drug loading amount: High Performance Liquid Chromatography (HPLC), with a device model of Agilent 1100.

**Table 1**

| | Particle size (nm) | Polydispersity index (PDI) | Surface charge (mV) | Drug loading amount % w/w |
|---|---|---|---|---|
| Example 1 | 204.2 ± 5.6 | 0.123 | -21 | 69% |
| Example 2 | 215.6 ± 3.7 | 0.154 | -26 | 70% |
| Example 3 | 234.2 ± 1.4 | 0.167 | -19 | 66% |
| Example 4 | 225.1 ± 0.9 | 0.103 | -12 | 61% |
| Example 5 | 250.4 ± 1.7 | 0.114 | -18 | 73% |
| Example 6 | 230.1 ± 4.6 | 0.133 | +47 | 68% |
| Example 7 | 204.2 ± 5.6 | 0.123 | -21 | 69% |
| Example 8 | 188.1 ± 2.3 | 0.158 | -18 | 60% |
| Comparative Example 1 | 204.2 ± 5.6 | 0.123 | -21 | 69% |

As can be seen from Table 1, in Examples 1 to 8, the nanocrystalline drugs have a relatively small particle size, a relatively uniform particle size distribution, and a relatively high drug loading amount. Further, the nanocrystalline drugs of Examples 1 to 5, 7, and 8 are negatively charged, so that it is possible to cooperate with the negatively charged microporous membrane, so as to promote the release of the crystalline drug on the drug coated balloon. The nanocrystalline drug of Example 6 is positively charged, so that it is possible to cooperate with the positive charged microporous membrane, so as to promote the release of the crystalline drug on the drug coated balloon.

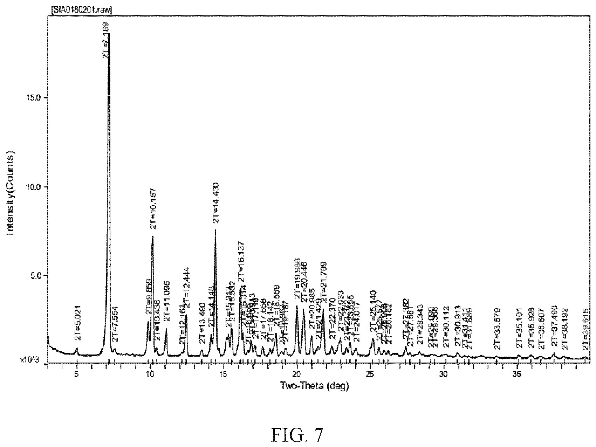

In addition, FIG. 6 is a graph illustrating a size distribution of the nanocrystalline drug prepared in Example 1, and FIG. 6 is an XRD pattern of the nanocrystalline drug prepared in Example 1. As can be seen from FIGS. 6 and 7, in Example 1, the nanocrystalline drug not only has a small particle size, but also has a high content of the crystalline drug, facilitating the slow-release effect of the drug coated balloon.

### Delivery Loss Test

The drug balloon prepared in the above examples is inserted into an in vitro vascular model, the time to reach the target is controlled to 60s, the drug balloon is not dilated, and then the drug balloon is taken out. The drug residue on the balloon surface is tested by High Performance Liquid Chromatography (HPLC), and the drug loss rate during delivery is calculated. The test results are shown in Table 2.

**Table 2**

| | Delivery loss % |
|---|---|
| Example 1 | 4% |
| Example 2 | 3% |
| Example 3 | 6% |
| Example 4 | 4% |
| Example 5 | 5% |
| Example 6 | 6% |
| Example 7 | 7% |
| Example 8 | 5% |
| Comparative Example 1 | 28% |

As can be seen from Table 2, the loss rate during delivery in Example 1 to 8 are low and are significantly lower than that in Comparative Example 1, which indicates that the crystalline drug on the drug-loaded implantable medical device of the present application is firmly bonded with the microporous membrane.

### Tissue Absorption Test

A segment of isolated porcine arterial blood vessel was kept at a constant temperature of 37°C. The porcine arterial blood vessel was dilated by a sterilized bare balloon for 1 min, at a pressure of 6 atm, and then the pressure was relieved and the sterilized bare balloon was taken out. The drug balloons prepared in the above Examples were placed into the dilated porcine arterial blood vessels to dilate the dilated porcine arterial blood vessels for 1 min, at a pressure of 6 atm, and then the pressure was relieved and the drug balloons were taken out. The porcine arterial blood vessels were immediately rinsed with phosphate buffered saline (PBS) 3 times with 1mL of PBS each time. Then, the drug concentrations in tissues are tested by Gas Chromatography-Mass Spectrometry (GC-MS), and the amount of drug residue on the surface of the balloon is tested by HPLC. The test results are shown in Table 3.

**Table 3**

| | Drug concentrations in tissues (ng/mg) | Drug residue on the surface of the balloon % |
|---|---|---|
| Example 1 | 349.2 ± 97 ng/mg | 4% |
| Example 2 | 417.8 ± 128 ng/mg | 3% |
| Example 3 | 477.1 ± 62 ng/mg | 6% |
| Example 4 | 560.4 ± 96 ng/mg | 8% |
| Example 5 | 305.3 ± 143 ng/mg | 6% |
| Example 6 | 644.3 ± 115 ng/mg | 4% |
| Example 7 | 211.8 ± 152 ng/mg | 22% |
| Example 8 | 368.8 ± 71 ng/mg | 7% |
| Comparative Example 1 | 102.3 ± 52 ng/mg | 17% |

As can be seen from Table 3, in Examples 1 to 6 and 8, the concentrations in the tissues are high, and the amounts of drug residues on the surfaces of the balloons are low, which is obviously advantageous over Comparative Example 1. In addition, it was found that the final amount of drug residue on the surface of the balloon in Example 7 is high, which indicates that the drug-loaded implantable medical device of the present application may more effectively release the drug when there is a charge repulsion effect in the tissue, so that the target tissue concentration can be achieved, and the drug utilization rate is high.

### Test for residence time in tissues

A segment of isolated porcine arterial blood vessel was kept at a constant temperature of 37°C. The porcine arterial blood vessel was dilated by a sterilized bare balloon for 1 min, at a pressure of 6 atm, and then the pressure was relieved and the sterilized bare balloon was taken out. The drug balloons prepared in the above Examples were placed into the dilated porcine arterial blood vessels to dilate the dilated porcine arterial blood vessels for 1 min, at a pressure of 6 atm, and then the pressure was relieved and the drug balloons was taken out. The porcine arterial blood vessels were immediately rinsed with phosphate buffered saline (PBS) 3 times with 1mL of PBS each time. Then, the porcine arterial blood vessels were placed and cultured in a culture medium for 7 days and 28 days, and in triplicate at each time point. After sampling, the drug concentrations in tissues were tested by Gas Chromatography-Mass Spectrometry (GC-MS). The test results were shown in Table 4.

**Table 4**

| | Drug concentrations in tissues (ng/mg) | |
|---|---|---|
| | 7 days | 28 days |
| Example 1 | 217.1 ± 82.4 | 74.2 ± 15.1 |
| Example 2 | 192.9 ± 68.8 | 99.5 ± 48.4 |
| Example 3 | 254.2 ± 82.5 | 114.8 ± 51.2 |
| Example 4 | 220.3 ± 73.1 | 122.1 ± 34.4 |
| Example 5 | 179.3 ± 51.2 | 65.1 ± 18.3 |
| Example 6 | 373.1 ± 112.8 | 187.2 ± 57.9 |
| Example 7 | 75.2 ± 34.7 | 0.6 ± 1.4 |
| Example 8 | 178.2 ± 22.6 | 80.2 ± 17.3 |
| Comparative Example 1 | 23.1 ± 14.3 | BQL |

BQL: below detection limit

As can be seen from Table 4, the coated balloons in Examples 1 to 8 exhibit an excellent slow-release effect, which is significantly advantageous over Comparative Example 1, and the slow-release effect of Examples 1 to 6, and 8 are advantageous over that of Example 7. It indicates that, the drug-loaded implantable medical device of the present application has an excellent slow-release effect. When the charge of the nanocrystalline drug is of the same type as the charge of the microporous membrane, the slow-release effect is better.

Each of the technical features of the above-mentioned embodiments may be combined arbitrarily. To simplify the description, not all the possible combinations of each of the technical features in the above embodiments are described. However, all of the combinations of these technical features should be considered as within the scope of this application, as long as such combinations do not contradict with each other.

The above embodiments merely illustrates several embodiments of the present application, and the description thereof is specific and detailed, but it shall not be constructed as limiting the scope of the present application. It should be noted that a plurality of variations and modifications may be made by those skilled in the art without departing from the scope of this application, which are all within the scope of protection of this application. Therefore, the protection scope of this application shall be subject to the appended claims.

## Claims

1. A drug-loaded implantable medical device, comprising a device body, a microporous membrane fixed on the device body, and a nanocrystalline drug loaded on a surface of the microporous membrane.

2. The drug-loaded implantable medical device of claim 1, wherein the surface of the microporous membrane and the nanocrystalline drug are charged, and a charge on the surface of the microporous membrane is of the same type as a charge on the nanocrystalline drug.

3. The drug-loaded implantable medical device of claim 1, wherein the microporous membrane is formed from at least one of nylon, polyvinylidene fluoride, mixed cellulose, polytetrafluoroethylene, polypropylene, polyethersulfone, or glass fibers.

4. The drug-loaded implantable medical device of claim 1, wherein the microporous membrane has a porosity in a range from 40% to 90%.

5. The drug-loaded implantable medical device of claim 1, wherein the microporous membrane has a pore size in a range from 0.02µm to 0.8µm.

6. The drug-loaded implantable medical device of claim 1, wherein the microporous membrane has a thickness in a range from 1µm to 200µm.

7. The drug-loaded implantable medical device of claim 1, further comprising a stabilizer adsorbed on a surface of the nanocrystalline drug, wherein the mass of the stabilizer is in a range from 0.2% to 20% of a total mass of the nanocrystalline drug.

8. The drug-loaded implantable medical device of claim 7, wherein the stabilizer is selected from at least one of poloxamer, polyvinylpyrrolidone (PVP), tween, hydroxypropyl methyl cellulose (HPMC), dextran, sodium dodecyl sulfate (SDS), sodium carboxymethylcellulose and polyvinyl alcohol (PVA).

9. The drug-loaded implantable medical device of claim 7, wherein the nanocrystalline drug is an anti-hyperplasia drug.

10. The drug-loaded implantable medical device of claim 1, wherein the nanocrystalline drug has a particle size in a range from 20nm to 300nm.

11. The drug-loaded implantable medical device of claim 1, wherein the nanocrystalline drug has a spherical, rod-like, worm-like or disk-like morphology.

12. The drug-loaded implantable medical device of claim 1, wherein in the nanocrystalline drug, a mass percentage of crystalline drugs is in a range from 70% to 100%.

13. The drug-loaded implantable medical device of claim 1, wherein the device body is a balloon.

14. A method for preparing a drug-loaded implantable medical device, comprising:
providing a microporous membrane;
loading a nanocrystalline drug on the microporous membrane; and
fixing the microporous membrane loaded with the nanocrystalline drug to the device body.

15. The method of claim 14, wherein the nanocrystalline drug is loaded on the microporous membrane by a mechanical filtration.

16. The method of claim 14, wherein the microporous membrane loaded with the nanocrystalline drug is fixed to the device body by a laser welding.

17. The method of claim 14, wherein the loading a nanocrystalline drug on the microporous membrane comprises:
dissolving a drug in a first solvent to obtain a drug solution;
suspending a stabilizer in a second solvent to obtain a stabilizer suspension;
adding the drug solution to the stabilizer suspension under stirring to obtain a mixed solution;
sonicating, and then dialyzing, concentrating the mixed solution to obtain a nanocrystalline drug suspension; and
loading the nanocrystalline drug in the nanocrystalline drug suspension on the microporous membrane, and then drying,
wherein one of the first solvent and the second solvent is an organic solvent that is miscible with water, and the other is water.
